# NOUVEAU FASCICULE DE BREVET EUROPEEN

(11) **EP 2 089 082 B2**
(45) Date de publication et mention de la décision concernant l'opposition: **22.02.2023**
(45) Mention de la délivrance du brevet: 20.05.2015
(21) Numéro de dépôt: 07821908.6
(22) Date de dépôt: 26.10.2007
(51) Int. Cl.: A61M 5/142, A61M 5/168, A61M 39/28

(54) **DISPOSITIF DE CONTRÔLE DE L'OUVERTURE OU LA FERMETURE D'UNE PINCE DANS UNE POMPE VOLUMÉTRIQUE**
VORRICHTUNG ZUR STEUERUNG DES ÖFFNENS UND SCHLIESSENS EINER KLAMMER BEI EINER VERDRÄNGERPUMPE
DEVICE FOR CONTROLLING THE OPENING AND CLOSING OF A CLAMP IN A POSITIVE DISPLACEMENT PUMP

(30) Priorité: 08.11.2006 FR 0609742
(43) Date de publication de la demande: 19.08.2009
(73) Titulaire: Fresenius Vial SAS, 38590 Brézins (FR)
(72) Inventeur: WOLFF, Rémy, 38210 Morette (FR)
(74) Mandataire: Maikowski & Ninnemann Patentanwälte Partnerschaft mbB
(86) Numéro de dépôt international: PCT/EP2007/061548
(87) Numéro de publication internationale: WO 2008/055793

(56) Documents cités:
- EP-A- 1 031 358
- EP-A2- 0 510 881
- WO-A-93/05829
- WO-A-98/56453
- WO-A-03/041787
- WO-A2-03/041787
- "Infusomat Space P", GEBRAUCHSANWEISUNG, January 2006 (2006-01-01)

## Description

L'invention concerne un procédé pour contrôler l'ouverture ou la fermeture d'une pince destinée à obturer un tube souple placé à l'intérieur d'un boîtier pouvant être fermé par une porte, la pince étant placée dans le boîtier et coopérant avec le tube pour l'ouvrir ou l'obturer. L'invention concerne également un dispositif de contrôle de l'ouverture ou de la fermeture d'une pince destinée à obturer un tube souple, notamment dans une pompe volumétrique, comprenant entre autres un boîtier pouvant être fermé par une porte, un tube placé à l'intérieur du boîtier ainsi qu'une pince placée dans le boîtier et coopérant avec le tube pour l'ouvrir ou l'obturer.

Il est très important dans le domaine de la perfusion qu'un tube reliant une source médicamenteuse à un patient via une pompe volumétrique ne se trouve jamais ouvert sans contrôle du débit.

Les systèmes de perfusion sont constitués en général d'une pompe volumétrique contenue dans un boîtier fermé par une porte, un tube souple traversant le boîtier en passant dans la pompe. Afin d'éviter que le liquide à perfuser commence à couler avant que le tube soit installé dans la pompe, il est courant de placer une pince en aval de la pompe. Cette pince est de préférence fermée durant toute l'opération de mise en place du tube dans la pompe avant d'être ouverte au moment de la perfusion. Il peut également y avoir un clamp à roulette en amont de la pompe.

Il est courant dans ce contexte de munir le système de moyens pour fermer la pince dès que la porte s'ouvre et des moyens pour ouvrir la pince dès que la porte se ferme. On citera par exemple la demande EP 0 238 227 A2 dans laquelle une pince coulissante est ouverte ou fermée par un organe associé à la poignée de la porte du boîtier de pompe. On retrouve le même principe dans le dispositif du document EP 1 218 055 A1 associé cette fois à une pince par compression.

La demande WO 03/041787 A2 présente une pompe d'infusion munie d'une pince escargot actionnée par une came à commande électrique. Cette came ferme la pince dès que la porte est ouverte.

Dans le document WO 93/05829 A1 on trouve une pince principale et une pince de sécurité. La première est constituée par une pince à glissière amovible présentant un partie large et une partie étroite, le tube pouvant passer de l'une à l'autre de sorte que dans la partie large le tube est ouvert et dans la partie étroite il est écrasé et donc en situation d'occlusion. La pince de sécurité, non amovible, est un élément à bascule qui dans la position redressée laisse le tube ouvert et en position basculée écrase le tube qui se trouve donc en situation d'occlusion. L'introduction de la pince dans la pompe, de même que son extraction hors de la pompe, provoque le glissement du tube dans la partie étroite. Par contre la fermeture de la porte provoque son ouverture. De même, la pince de sécurité est ouverte lorsque la porte arrive dans sa position fermée, mais se referme automatiquement dès que la porte est ouverte.

Dans le document WO 98/56453 A1, on trouve une pompe munie également de deux pinces. La première est une pince à glissière amovible qui est placée en position fermée lorsque la porte est ouverte et en position ouverte lorsque la porte est fermée. La deuxième, non amovible, est une pince de sécurité qui tend à se redresser en fermant le tube lorsque la porte est ouverte, mais qui peut être rabattue (manuellement ou par la porte) pour être mise en position ouverte.

L'ouverture de la pince lorsque la porte passe en position fermée peut cependant s'avérer dangereuse, notamment si le tube n'est pas correctement placé dans la pompe ou si la pompe a un défaut. Dans ce cas, il risque de se produire un écoulement incontrôlé du liquide de perfusion en direction du patient avec tous les risques que cela entraîne.

De même, on connaît, par exemple de la demande EP 1 031 358 A1, un système de pompe dans lequel des moyens sont prévus pour fermer le tube alors que la pompe est déjà allumée et le tube placé dans la pompe, pour permettre un contrôle de l'intégrité du système. Cette fermeture de la pince se fait de façon électrique alors que la porte est fermée. Cependant, ce système présente de nombreux inconvénients. Si la pompe est éteinte, si la batterie est trop faible ou si le moteur pour fermer la pince est hors service par exemple, cette dernière ne peut pas être fermée. De plus, il ne permet pas de fermer de façon ciblée la pince lorsque la porte de la pompe est actionnée.

Le document WO 03/041787 A2 déjà évoqué propose également un tel contrôle d'intégrité. Pour cela, un programme de contrôle est mis en marche une fois que la porte a été fermée. La commande électrique fait tourner la came qui à son tour ferme la pince à escargot. Cette pompe présente les mêmes inconvénient que ceux évoqués précédemment. On connaît aussi le document EP-A-0510881 A2.

L'objectif de l'invention est de développer un procédé et un dispositif de sécurité du type évoqué précédemment qui permette indépendamment de l'état de la batterie, de l'état allumé ou éteint de la pompe ou du bon positionnement du tube dans la pompe, d'empêcher un écoulement incontrôlé du liquide de perfusion dans le tube même après la fermeture de la porte de la pompe.

Cet objectif est atteint conformément à l'invention par le procédé et le dispositif de l'invention. Pour cela, le procédé prévoit de fermer la pince lorsque la fermeture de la porte est provoquée. Dans le dispositif, le système de sécurité comprend en outre des moyens pour fermer la pince lorsque la fermeture de la porte est provoquée. Ces moyens permettent de fermer la pince si elle est en position ouverte tout en la laissant fermée si elle est déjà dans cette position. Ainsi, le liquide de perfusion ne peut pas s'écouler librement après la fermeture de la porte.

Dans l'invention, il est prévu de fermer également la pince lorsque l'ouverture de la porte est provoquée. Pour cela, le dispositif de contrôle est muni de moyens pour fermer la pince lorsque l'ouverture de la porte est provoquée.

Les moyens pour fermer la pince lorsque l'ouverture de la porte est provoquée et les moyens pour fermer la pince lorsque la fermeture de porte est provoquée sont identiques. Ainsi, les mêmes moyens assureront la fermeture de la pince aussi bien au moment de l'ouverture de la porte qu'au moment de sa fermeture.

Les moyens pour fermer la pince lorsque l'ouverture de la porte est provoquée et les moyens pour fermer la pince lorsque la fermeture de la porte est provoquée sont actionnés par des moyens pour provoquer l'ouverture ou la fermeture de la porte. On pourra par exemple prévoir que les moyens pour ouvrir ou fermer la porte comprennent une poignée actionnable de l'extérieur du boîtier.

Une solution simple consiste à concevoir les moyens pour fermer la pince lorsque l'ouverture de la porte est provoquée et/ou les moyens pour fermer la pince lorsque la fermeture de la porte est provoquée sous la forme d'une came pouvant coopérer avec les moyens pour ouvrir ou fermer la porte. Il est préférable que la came soit solidaire de l'axe de la poignée.

On peut utiliser comme pince par exemple une pince coulissante, couramment appelée slide-clamp, constituée d'une partie fixe et d'une partie mobile, de préférence en rotation, les moyens pour fermer la pince lorsque l'ouverture de la porte est provoquée ou les moyens pour fermer la pince lorsque la fermeture de la porte est provoquée étant dimensionnés pour provoquer le glissement de la partie mobile du slide-clamp par rapport à la partie fixe.

Il est préférable de prévoir des moyens pour provoquer l'ouverture de la pince après que la porte a été fermée. On pourra à cet effet prévoir des moyens de commande pour actionner les moyens d'ouverture de la pince lorsqu'un certain événement a eu lieu. Cet événement pourra être par exemple la réalisation d'un test d'intégrité du système.

Afin d'éviter que la pince ne soit ouverte au moment précis où l'ouverture de la porte est provoquée, il est préférable de prévoir un fusible mécanique pour désactiver les moyens d'ouverture de la pince au cas où l'ouverture de la porte serait provoquée au moment où les moyens d'ouverture sont actionnés.

Ce dispositif de sécurité est tout particulièrement destiné à une pompe volumétrique raccordée au tube, de préférence une pompe péristaltique, la pompe volumétrique étant placée de préférence en amont de la pince

Un exemple de réalisation de l'invention est décrit ci-dessous à l'aide des figures qui montrent :
- Figure 1 :: Une vue en coupe à travers le boîtier de la pompe, au moment où la porte est fermée, ce qui provoque la fermeture de la pince ;
- Figure 2 :: Une vue en coupe selon la figure 1, la porte est fermée et la poignée relâchée, la pince reste fermée ;
- Figure 3 :: Une vue en coupe selon la figure 1, la pince est ouverte par le dispositif d'ouverture ;
- Figure 4 :: Une vue en coupe selon la figure 1, au moment où la porte va être ouverte, la poignée étant soulevée, ce qui provoque la fermeture de la pince ;
- Figure 5 :: Une vue en coupe selon la figure 1, dans laquelle le fusible mécanique a sauté lorsqu'on a tenté d'ouvrir la porte au moment où la pince allait être ouverte selon la figure 3 ;
- Figure 6 :: Une vue éclatée en perspective des éléments de la pince ;
- Figure 7 :: Une vue en perspective de la porte ;
- Figure 8 :: Une autre vue en perspective de la porte ;
- Figure 9 :: Une vue en coupe du fusible mécanique en position de fonctionnement ; et
- Figure 10 :: Une vue en coupe selon la figure 9 du fusible mécanique ayant sauté.

Le dispositif de l'invention se compose essentiellement d'un tube (1) passant dans une pompe volumétrique non représentée et pouvant être fermé ou ouvert par une pince (2). La pompe ainsi que la pince (2) sont placées dans un boîtier (3) pouvant être fermé par une porte (4).

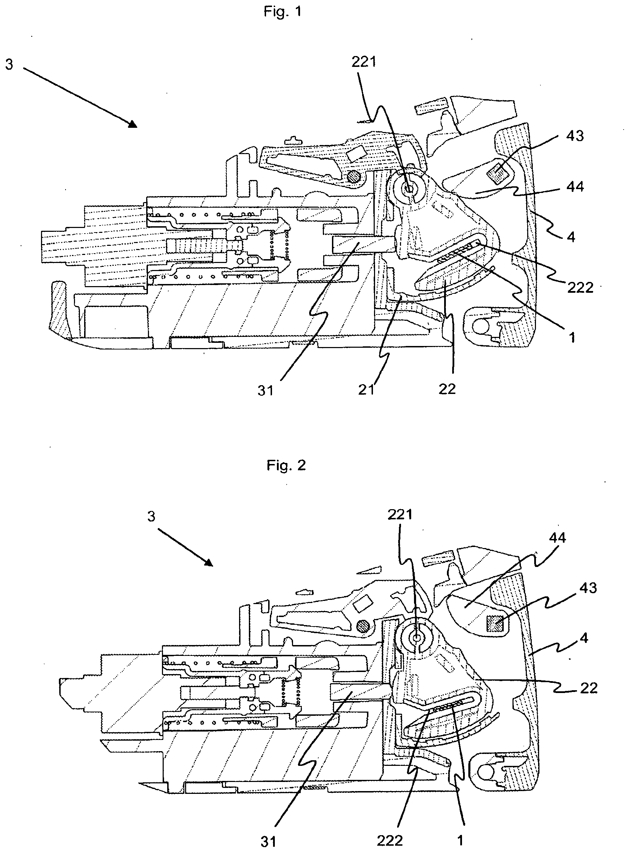

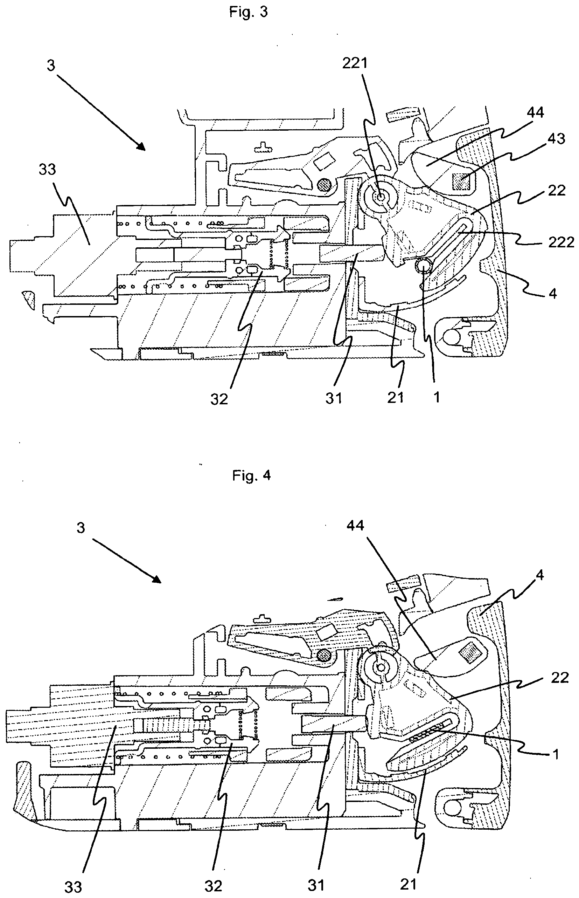

Dans l'exemple présenté ici, on utilise une pince dite slide-clamp se composant de deux parties : une première partie fixe (21) et une partie mobile (22). Le tube (1) passe à travers une ouverture sensiblement cylindrique (211) qui a pour fonction notamment de le maintenir en place radialement. Cette partie fixe est solidaire du boîtier (3) et ne bouge pas. La partie mobile (22) au contraire peut pivoter autour d'un pivot (221) qui peut être logé par exemple dans la partie fixe (21) de la pince (2). Cette partie mobile (22) est munie d'une fente (222) s'ouvrant sur une partie (223) plus large. La largeur de la fente (222) est choisie de telle sorte que le tube (1) est aplati lorsqu'il se trouve engagé dedans se trouvant ainsi obstrué. Au contraire, l'ouverture (223) est suffisamment large pour que le tube (1) soit presque libre de toute contrainte lorsqu'il se trouve dedans de sorte qu'il retrouve sensiblement sa forme cylindrique normale laissant le passage au liquide pompé. Dans la pratique, le tube est maintenu dans une forme légèrement pincée qui facilite son entrée dans la fente (222). L'ouverture (223) est cependant suffisamment large pour ne pas perturber l'écoulement du liquide. Une section de transition (224) permet de faire passer le tube de la position libre de toute contrainte dans l'ouverture (223) à la position aplatie dans la fente (222). Cette partie mobile (22) de la pince (2) peut donc pivoter d'une position d'ouverture dans laquelle son ouverture (223) est sensiblement alignée avec l'ouverture (211) de la partie fixe, position dans laquelle le tube (1) est ouvert, à une position de fermeture dans laquelle l'ouverture (211) de la partie fixe est alignée avec la fente (222) de la partie mobile, position dans laquelle le tube aplati est fermé. La figure 1 par exemple montre la position fermée tandis que la figure 3 montre la position ouverte.

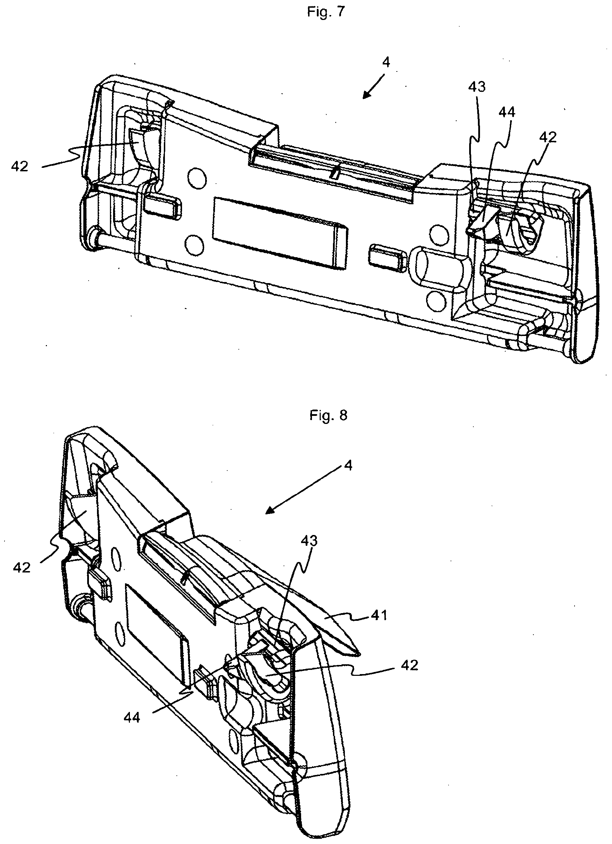

La fermeture de la pince (2) est assurée par des moyens de fermeture fixés sur la porte (4). La porte (4) est munie d'une poignée (41) devant être pivotée pour permettre l'ouverture ou la fermeture de la porte (4). En faisant pivoter la poignée (41), on provoque le pivotement de crochets (42). Lorsque la poignée est en position rabattue, les crochets (42) crochètent des saillies correspondantes du boîtier (3) non représentées ici en empêchant l'ouverture involontaire de la porte (4). Quand la poignée (41) est en position pivotée, les crochets (42) se décrochent des saillies permettant l'ouverture de la porte (4). Pour ce faire, la poignée est montée sur un axe (43) sur lequel sont également montés les crochets (42). Outre ces crochets (42) nécessaires à l'ouverture et la fermeture de la porte, l'axe (43) porte également une came (44). Cette came (44) peut donc elle aussi pivoter entre une position pivotée où elle provoque lorsque la porte (4) est fermée le pivotement en position fermée de la partie mobile (22) de la pince (2), et une position rabattue dans laquelle elle ne bloque plus la partie mobile (22) en position fermée. La position pivotée de la came (44) est visible sur les figures 1, 4, 7 et 8, tandis que la position rabattue est visible sur les figures 2 et 3. La came (44) joue donc le rôle de moyens de fermeture aussi bien pour fermer la pince lorsque la fermeture de la porte est provoquée que pour fermer la pince lorsque l'ouverture de la porte est provoquée. De même, l'axe (43) et la poignée (41) constituent les moyens pour provoquer l'ouverture ou la fermeture de la porte (4).

L'ouverture de la pince (2) est assurée par un doigt mobile (31) placé dans le boîtier (3) et actionné par un moteur (33). Ce doigt (31) en se déplaçant en translation vient appuyer sur la face de la partie mobile (22) de la pince (2) opposée à celle contre laquelle appuie la came (44). Ceci provoque le pivotement de la partie mobile (22) de sa position fermée vers sa position ouverte. Ce doigt mobile (31) associé au moteur (33) constitue les moyens pour provoquer l'ouverture de la pince (2) après que la porte a été fermée.

Le mode de fonctionnement du dispositif de contrôle selon l'invention est décrit ci-dessous en référence aux figures 1 à 4. Pour fermer la porte (4), il faut soulever la poignée (41) afin que les crochets (42) puissent contourner les saillies de fermeture. En soulevant la poignée, on provoque le basculement de la came (44). Lorsque la porte est poussée vers sa position fermée, la came ainsi basculée vient appuyer sur la face de la partie mobile (22) de la pince située du côté du fond de la fente (222). Ceci provoque le pivotement de la partie mobile (22) autour de son axe (221) forçant le tube (1) à pénétrer dans la fente (222). Ce dernier, aplati dans la fente, se trouve donc en position fermée. C'est la position qui est représentée à la figure 1.

Dès que la poignée est rabattue, la came (44) et les crochets (43) retournent dans leurs positions rabattues respectives comme le montre la figure 2. La longueur de la fente (222) est telle que la partie mobile (22) ne peut pas retourner sans aide extérieure en position ouverte.

Le dispositif se trouve donc avec la porte (4) fermée et le tube (1) occlus. Il est maintenant possible par exemple de réaliser un test d'intégrité de la pompe sans que le liquide ne soit administré au patient. Si le résultat du test est favorable, le doigt (31) est actionné provoquant le pivotement en position ouverte de la partie mobile (22) de la pince (2). La poignée (4) étant rabattue, la came (44) n'entrave pas ce mouvement de pivot. On se retrouve dans la position présentée à la figure 3. Une fois le mouvement de pivot achevé, le doigt (31) retourne dans sa position initiale qui n'entrave pas le mouvement de pivotement inverse provoqué par la came (44).

Si pour une raison quelconque, la porte doit être ouverte, il faut tout d'abord faire pivoter la poignée (41) provoquant d'une part le pivotement des crochets (43) pour qu'ils se décrochent des saillies et d'autre part le pivotement de la came (44) ce qui provoque à son tour le pivotement de la partie mobile (22) de la pince (2). On se retrouve dans la position présentée à la figure 4.

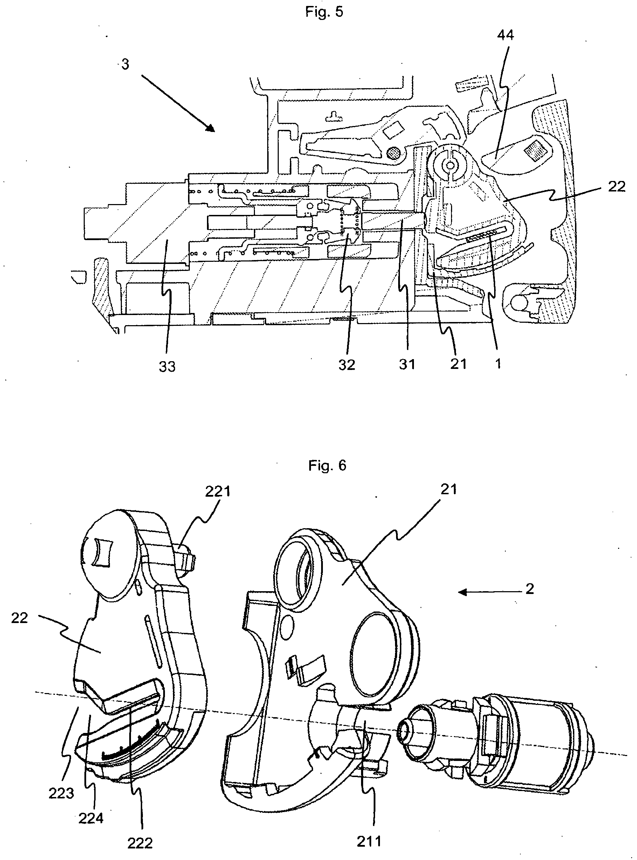

Il est impératif que la pince soit en position fermée lorsque la porte est ouverte. Autrement dit, si pour une raison quelconque, quelqu'un ouvre la porte au moment précis où le doigt (31) se déplace en translation pour provoquer le pivotement de la partie mobile (22) en position ouverte, il faut que le mouvement de fermeture de la pince (2) ait la priorité sur le mouvement inverse d'ouverture. De même, si l'ouverture est provoquée lorsque le doigt (31) est en position sortie, il faut pouvoir tout de même fermer la pince (2). Pour cela, il est prévu d'intercaler entre le moteur et le doigt (31) un fusible mécanique (32) qui cède si la pression à exercer sur le doigt (31) pour le déplacer en translation est supérieure à une valeur normale. La figure 5 montre un tel exemple. On voit que la came (44) est en position pivotée empêchant la partie mobile (22) de retourner en position ouverte. Le fusible mécanique (32) a alors joué son rôle en cédant de sorte que le moteur n'a pas pu entraîner en translation le doigt (31).

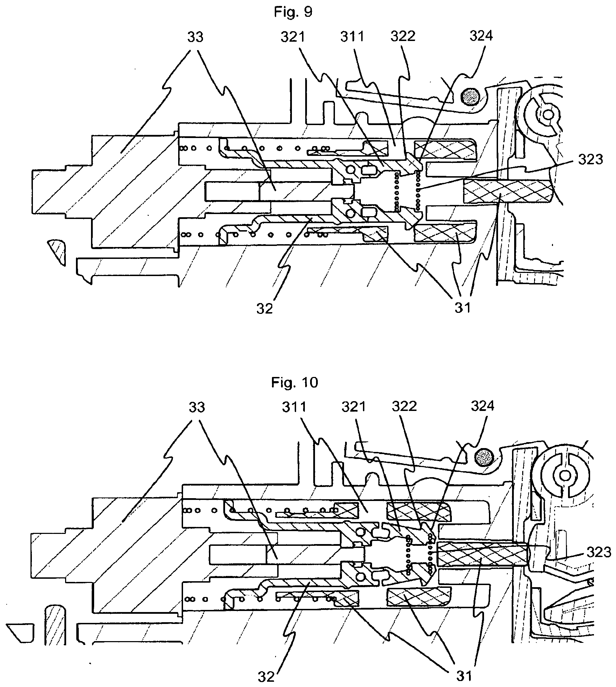

Le mode de fonctionnement du fusible mécanique (32) est expliqué plus en détail aux figures 9 et 10. Le fusible (32) est muni de crochets (321) qui sont maintenus dans leur position de travail visible à la figure 9 par un ressort (323) qui tend à les repousser radialement vers l'extérieur. Ces crochets (321) pénètrent normalement dans des encoches (311) réalisées dans le doigt (31). Ainsi, lorsque le moteur (33) provoque un mouvement de rétractation vers la droite, il entraîne le fusible (32) et avec lui les crochets (321). Ceux-ci entraînent à leur tour le doigt (31) grâce aux butées (322) situées à leurs sommets qui s'appuient contre l'extrémité arrière (à gauche sur la figure) des encoches (311). Ceci est bien visible à la figure 4. Si par contre le moteur (33) provoque un mouvement de translation vers la droite, il entraîne le fusible (32) et avec lui les crochets (321) qui viennent buter contre l'autre extrémité des encoches (311) du doigt (31) en l'entraînant dans ce mouvement général de sortie vers la droite. C'est ce que montrent les figures 3 et 9.

Si la came (44) bloque le mouvement de la partie pivotante (22) de la pince (2), comme dans le cas de la figure 5, les crochets (321) cèdent alors et pivotent vers le centre du fusible (32) contre l'effet du ressort (323) grâce à des biseaux (324) situés à leurs sommets sur la face opposée aux butées (322). Le fusible se retrouve dans la position présentée aux figures 5 et 10. Les moyens d'ouverture, constitués par le doigt (31), sont ainsi désactivés et ils ne peuvent plus provoquer l'ouverture de la pince (2).

De la même manière, si quelqu'un ouvre la porte (4) alors que le doigt (31) est encore sorti et la pince (2) en position ouverte comme sur la figure 3, cela provoque le pivotement de la came (44) qui vient rabattre en position fermée la partie pivotante (22) de la pince (2). Celle-ci à son tour pousse le doigt (31). Le fusible (32) ne pouvant pas reculer puisque le moteur (33) n'est pas en position rétractée, ce sont les crochets (321) qui cèdent en pivotant vers le centre du fusible (32) contre l'effet du ressort (322). C'est la position présentée aux figures 6 et 10. Le fusible a donc sauté. Les moyens d'ouverture (31) sont à nouveau désactivés et ne peuvent plus assurer l'ouverture de la pince (2).

Une fois la porte refermée, le fusible peut se réarmer automatiquement dès que le moteur (33) retourne dans sa position rétractée présentée par exemple à la figure 1. Le ressort (323) repousse les crochets (321) dans les encoches (311) du doigt (31) dès que le fusible a suffisamment reculé.

L'exemple de réalisation présenté ici fait appel à une pince dans laquelle le tube est pincé dans une fente pouvant pivoter. Il va de soi que la fente peut aussi avoir un mouvement de translation plutôt qu'un mouvement de pivot. De même, il est tout aussi bien possible d'utiliser une pince escargot (également connue sous le nom de pinch clamp) entre deux butées mobiles l'une par rapport à l'autre, que ce soit par translation ou par pivotement.

Le mouvement du doigt (31) ne doit pas être nécessairement une translation ; il peut très bien s'agir d'un mouvement de pivotement par exemple. De même, le moteur électrique peut être remplacé par une action manuelle ou un dispositif mécanique.

Avec le dispositif de sécurité de l'invention, on s'assure que le tube est non seulement toujours fermé lorsque la porte est en position ouverte mais également qu'il reste fermé une fois la porte fermée tant qu'un dispositif approprié n'a pas provoqué l'ouverture de la pince. Ce dispositif de fermeture entièrement mécanique n'est pas soumis aux aléas d'une alimentation électrique éventuellement défectueuse.

### Références :

1 Tube
2 Pince
   21 Partie fixe
      211 Ouverture cylindrique
   22 Partie mobile
      221 Pivot
      222 Fente
      223 Ouverture
      224 Section de transition
3 Boîtier de la pompe
   31 Doigt
      311 Encoches
   32 Fusible mécanique
      321 Crochets
      322 Butée
      323 Ressort
      324 Biseau
   33 Moteur
4 Porte
   41 Poignée
   42 Crochets
   43 Axe de pivotement de la poignée
   44 Came

## Revendications

1. Procédé pour contrôler l'ouverture ou la fermeture d'une pince (2) destinée à obturer un tube souple (1) placé à l'intérieur d'un boîtier (3) pouvant être fermé par une porte (4), la pince (2) étant placée dans le boîtier (3) et coopérant avec le tube (1) pour l'ouvrir ou l'obturer, dans lequel la pince (2) passe d'une position d'ouverture à une position de fermeture lorsque la fermeture de la porte (4) est provoquée et reste dans cette position de fermeture une fois la porte fermée tant que des moyens d'ouverture n'ont pas provoqué l'ouverture de la pince,
**caractérisé en ce que**
la pince (2) passe de la position d'ouverture à la position de fermeture lorsque l'ouverture de la porte (4) est provoquée, dans lequel des moyens (44) pour fermer la pince (2) lorsque l'ouverture de la porte (4) est provoquée et des moyens (44) pour fermer la pince (2) lorsque la fermeture de porte (4) est provoquée sont identiques,
dans lequel les moyens (44) pour fermer la pince (2) lorsque l'ouverture de la porte (4) est provoquée et les moyens (44) pour fermer la pince (2) lorsque la fermeture de la porte (4) est provoquée sont actionnés par des moyens (41, 43) pour provoquer l'ouverture ou la fermeture de la porte (4).

2. Procédé selon la revendication 1, **caractérisé en ce que** la pince (2) n'est rouverte une fois la porte fermée qu'après qu'un événement prédéterminé a eu lieu, notamment après qu'un test d'intégrité a été réalisé avec succès.

3. Procédé selon la revendication 2, **caractérisé en ce que** la réouverture de la pince (2) n'a pas lieu si au même moment l'ouverture de la porte (4) est provoquée.

4. Dispositif de contrôle de l'ouverture ou de la fermeture d'une pince (2) destinée à obturer un tube souple (1), notamment dans une pompe volumétrique, comprenant entre autres
- un boîtier (3) pouvant être fermé par une porte (4),
- un tube (1) placé à l'intérieur du boîtier (3), et
- une pince (2) placée dans le boîtier (3) et coopérant avec le tube (1) pour l'ouvrir ou l'obturer,
dans lequel des moyens (44) sont prévus pour faire passer la pince (2) d'une position d'ouverture à une position de fermeture lorsque la fermeture de la porte (4) est provoquée et en ce des moyens (31) sont prévus pour provoquer l'ouverture de la pince (2) après que la porte (4) a été fermée,
**caractérisé en ce que**
des moyens (44) sont prévus pour faire passer la pince (2) d'une position d'ouverture à une position de fermeture lorsque l'ouverture de la porte (4) est provoquée, dans lequel les moyens (44) pour fermer la pince (2) lorsque l'ouverture de la porte (4) est provoquée et les moyens (44) pour fermer la pince (2) lorsque la fermeture de porte (4) est provoquée sont identiques,
dans lequel les moyens (44) pour fermer la pince (2) lorsque l'ouverture de la porte (4) est provoquée et les moyens (44) pour fermer la pince (2) lorsque la fermeture de la porte (4) est provoquée sont actionnés par des moyens (41, 43) pour provoquer l'ouverture ou la fermeture de la porte (4).

5. Dispositif selon la revendication 4, **caractérisé en ce que** les moyens pour provoquer l'ouverture ou la fermeture de la porte comprennent une poignée (41) actionnable de l'extérieur du boîtier (3).

6. Dispositif selon la revendication 4 ou 5, **caractérisé en ce que** les moyens (44) pour fermer la pince (2) lorsque la fermeture de la porte (4) est provoquée et/ou les moyens (44) pour fermer la pince (2) lorsque l'ouverture de la porte (4) est provoquée comprennent une came (44) pouvant coopérer avec les moyens (41, 43) pour provoquer l'ouverture ou la fermeture de la porte (4).

7. Dispositif selon la revendication 6, **caractérisé en ce que** la came est solidaire de l'axe (43) de la poignée (41).

8. Dispositif selon l'une des revendications 4 à 7, **caractérisé en ce que** la pince (2) est constituée par un slide-clamp constituée d'une partie fixe (21) et d'une partie mobile (22), de préférence en rotation, les moyens pour fermer la pince (2) lorsque la fermeture de la porte est provoquée ou les moyens pour fermer la pince (2) lorsque l'ouverture de la porte est provoquée étant dimensionnés pour provoquer le glissement de la partie mobile (22) du slide-clamp par rapport à la partie fixe (21).

9. Dispositif selon la revendication 4 à 8, **caractérisé en ce que** des moyens de commande (33) sont prévus pour actionner les moyens d'ouverture (31) de la pince (2) lorsqu'un certain événement a eu lieu.

10. Dispositif selon la revendication 9, **caractérisé en ce que** les moyens de commande sont conçus pour actionner les moyens d'ouverture (31) de la pince (2) après qu'un test d'intégrité du système a été réalisé.

11. Dispositif selon l'une des revendications 8 à 10, **caractérisé en ce qu'**un fusible mécanique (32) est prévu pour désactiver les moyens d'ouverture (31) de la pince (2) au cas où l'ouverture de la porte serait provoquée au moment où les moyens d'ouverture sont actionnés.

12. Dispositif selon l'une des revendications 4 à 11, **caractérisé en ce qu'**il comprend en outre une pompe volumétrique raccordée au tube (1), de préférence une pompe péristaltique, la pompe volumétrique étant placée de préférence en amont de la pince (2).

## Patentansprüche

1. Verfahren zum Kontrollieren des Öffnens oder Schließens einer Klemme (2), die zum Verschließen eines flexiblen Schlauches (1) ausgebildet ist, der im Innern eines Gehäuses (3) angeordnet ist, das durch eine Tür (4) verschließbar ist, wobei die Klemme (2) in dem Gehäuse (3) angeordnet ist und mit dem Schlauch (1) zusammenwirkt, um ihn freizugeben oder zu verschließen, wobei die Klemme (2) von einer geöffneten in eine geschlossene Stellung übergeht, wenn das Schließen der Tür ausgelöst wird und nach dem Schließen der Tür in dieser geschlossenen Stellung verbleibt, solange Öffnungsmittel nicht das Öffnen der Klemme ausgelöst haben,
**dadurch gekennzeichnet,**
**dass** die Klemme (2) von der geöffneten Stellung in die geschlossene Stellung übergeht, wenn das Öffnen der Tür (4) ausgelöst wird, wobei Mittel (44) zum Schließen der Klemme (2), wenn das Öffnen der Tür (4) ausgelöst wird und Mittel (44) zum Schließen der Klemme (2), wenn das Schließen der Tür (4) ausgelöst wird, identisch sind,
wobei die Mittel (44) zum Schließen der Klemme (2), wenn das Öffnen der Tür ausgelöst wird, und die Mittel (44) zum Schließen der Klemme (2), wenn das Schließen der Tür (4) ausgelöst wird, durch Mittel (41, 43) zum Auslösen des Öffnens oder des Schließens der Tür (4) betätigt werden.

2. Verfahren gemäß Anspruch 1, **dadurch gekennzeichnet, dass** die Klemme (2) nach dem Schließen der Tür erst dann geöffnet wird, wenn ein vorherbestimmtes Ereignis stattgefunden hat, insbesondere nachdem ein Integritätstest erfolgreich durchgeführt wurde.

3. Verfahren gemäß Anspruch 2, **dadurch gekennzeichnet, dass** das Wiederöffnen der Klemme (2) nicht erfolgt, wenn gleichzeitig das Öffnen der Tür (4) ausgelöst wird.

4. Vorrichtung für die Kontrolle des Öffnens oder Schließens einer Klemme (2), die zum Verschließen eines flexiblen Schlauches (1), insbesondere in einer Volumenpumpe ausgebildet ist, die unter anderem umfasst:
- ein Gehäuse (3), das durch eine Tür (4) verschließbar ist,
- einen Schlauch (1), der in dem Gehäuse (3) angeordnet ist, und
- eine Klemme (2), die in dem Gehäuse (3) angeordnet ist und mit dem Schlauch (1) zusammenwirkt, um ihn zu freizugeben oder zu verschließen,
wobei Mittel (44) vorgesehen sind, um die Klemme (2) von einer geöffneten Stellung in eine geschlossene Stellung zu überführen, wenn das Schließen der Tür (4) ausgelöst wird und dass Mittel (31) vorgesehen sind, um das Öffnen der Klemme (2) auszulösen, nachdem die Tür (4) geschlossen wurde,
**dadurch gekennzeichnet,**
**dass** Mittel (44) vorgesehen sind, um die Klemme (2) von einer geöffneten Stellung in eine geschlossene Stellung zu überführen, wenn das Öffnen der Tür (4) ausgelöst wird, wobei die Mittel (44) zum Schließen der Klemme (2), wenn das Öffnen der Tür (4) ausgelöst wird, und die Mittel (44) zum Schließen der Klemme (2), wenn das Schließen der Tür (4) ausgelöst wird, identisch sind,
wobei die Mittel (44) zum Schließen der Klemme (2), wenn das Öffnen der Tür ausgelöst wird und die Mittel (44) zum Schließen der Klemme (2), wenn das Schließen der Tür (4) ausgelöst wird, durch Mittel (41, 43) zum Auslösen des Öffnens oder des Schließens der Tür (4) betätigt werden.

5. Vorrichtung gemäß Anspruch 4, **dadurch gekennzeichnet, dass** die Mittel zum Auslösen des Öffnens oder des Schließens der Tür einen Türgriff (41) umfassen, der von der Außenseite des Gehäuses (3) betätigbar ist.

6. Vorrichtung gemäß Anspruch 4 oder 5, **dadurch gekennzeichnet, dass** die Mittel (44) zum Schließen der Klemme (2), wenn das Schließen der Tür (4) ausgelöst wird, und/oder die Mittel (44) zum Schließen der Klemme (2), wenn das Öffnen der Tür (4) ausgelöst wird, eine Nocke (44) umfassen, die mit den Mitteln (41, 43) zum Auslösen des Öffnens oder Schließens der Tür (4) zusammenwirken kann.

7. Vorrichtung gemäß Anspruch 6, **dadurch gekennzeichnet, dass** die Nocke mit der Achse (43) des Türgriffs (41) fest verbunden ist.

8. Vorrichtung gemäß einem der Ansprüche 4 bis 7, **dadurch gekennzeichnet, dass** die Klemme (2) aus einer Slide-Clamp besteht, die ein festes Teil (21) und ein bewegliches, vorzugsweise drehbewegliches, Teil (22) aufweist, wobei die Mittel zum Schließen der Klemme (2), wenn das Schließen der Tür ausgelöst wird oder die Mittel zum Schließen der Klemme (2), wenn das Öffnen der Tür ausgelöst wird so dimensioniert sind, das sie das Gleiten des beweglichen Teils (22) der Slide-Clamp relativ zum festen Teil (21) verursachen.

9. Vorrichtung gemäß den Patentansprüchen 4 bis 8, **dadurch gekennzeichnet, dass** Steuermittel (33) vorgesehen sind, um die Mittel (31) zum Öffnen der Klemme (2) betätigen, wenn ein bestimmtes Ereignis stattgefunden hat.

10. Vorrichtung gemäß Anspruch 9, **dadurch gekennzeichnet, dass** die Steuermittel ausgebildet sind, um die Mittel (31) zum Öffnen der Klemme (2) zu betätigen, nachdem ein Integritätstest des Systems durchgeführt wurde.

11. Vorrichtung gemäß einem der Ansprüche 8 bis 10, **dadurch gekennzeichnet, dass** eine mechanische Sicherung (32) vorgesehen ist, um die Mittel (31 zum Öffnen der Klemme (2) zu inaktivieren, wenn das Öffnen der Tür in dem Moment erfolgt, in dem die Mittel zum Öffnen betätigt werden.

12. Vorrichtung gemäß einem der Ansprüche 4 bis 11, **dadurch gekennzeichnet, dass** sie weiterhin eine volumetrische Pumpe, vorzugsweise eine peristaltische Pumpe umfasst, die mit dem Schlauch (1) verbunden ist, wobei die volumetrische Pumpe vorzugsweise strömungsaufwärts der Klemme (2) angeordnet ist.

## Claims

1. Method for controlling the opening or closing of a clamp (2) intended to seal a flexible tube (1) placed inside a housing (3) that can be closed with a door (4), the clamp (2) being placed in the housing (3) and cooperating with the tube (1) in order to open or seal it, wherein the clamp (2) passes from an open position to a closed position when the closing of the door is triggered, and stays in this closed position after closing of the door as long as opening means did not trigger the opening of the clamp,
**characterized in**
**that** the clamp (2) passes from the open position to the closed position when the opening of the door (4) is triggered, wherein means (44) for closing the clamp (2), when the opening of the door (4) is triggered, and means (44) for closing the clamp (2), when the closing of the door (4) is triggered, are identical,
wherein the means (44) for closing the clamp (2), when the opening of the door is triggered, and the means (44) for closing the clamp (2), when the closing of the door (4) is triggered, are activated by means (41, 43) for triggering the opening or the closing of the door (4).

2. Method according to claim 1, **characterized in that**, once the door is closed, the clamp (2) is only opened when a predetermined event has occurred, in particular after an integrity test has been successfully conducted.

3. Method according to claim 2, **characterized in that** the reopening of the clamp (2) is not effected when the opening of the door (4) is triggered at the same time.

4. Device for controlling the opening or closing of a clamp (2) intended to seal a flexible tube (1), in particular in a positive displacement pump, comprising, among other things:
- a housing (3) that can be closed with a door (4),
- a tube (1) that is placed inside the housing (3), and
- a clamp (2) that is placed inside the housing (3) and cooperates with the tube (1) in order to open or seal it,
wherein means (44) are provided for passing the clamp (2) from an open position to a closed position when the closing of the door (4) is triggered, and wherein means (31) are provided for triggering the opening of the clamp (2) after the door (4) has been closed,
**characterized in**
**that** means (44) are provided for passing the clamp (2) from an open position to a closed position when the opening of the door (4) is triggered, wherein the means (44) for closing the clamp (2), when the opening of the door (4) is triggered, and the means (44) for closing the clamp (2), when the closing of the door (4) is triggered, are identical,
wherein the means (44) for closing the clamp (2), when the opening of the door is triggered, and the mans (44) for closing the clamp (2), when the closing of the door (4) is triggered, are activated by means (41, 43) for triggering the opening or the closing of the door (4).

5. Device according to claim 4, **characterized in that** the means for triggering the opening or the closing of the door comprise a door handle (41) that is operable from the outside of the housing (3).

6. Device according to claim 4 or 5, **characterized in that** the means (44) for closing the clamp (2), when the closing of the door (4) is triggered, and/or the means (44) for closing the clamp (2), when the opening of the door (4) is triggered, comprise a cam (44) that is able to cooperate with the means (41, 43) for triggering the opening or closing of the door (4).

7. Device according to claim 6, **characterized in that** the cam is firmly connected to the axle (43) of the door handle (41).

8. Device according to any of claims 4 to 7, **characterized in that** the clamp (2) consists of a slide clamp, which includes a stationary part (21) and a mobile part (22), preferably with rotational mobility, wherein the means for closing the clamp (2), when the closing of the door is triggered, or the means for closing the clamp (2), when the opening of the door is triggered, are dimensioned so as to make the mobile part (2) of the slide clamp slide relative to the stationary part (21).

9. Device according to claims 4 to 8, **characterized in that** control means (33) are provided in order to activate the means (31) for opening the clamp (2) when a certain event has occurred.

10. Device according to claim 9, **characterized in that** the control means are designed so as to activate the means (31) for opening the clamp (2) after a system integrity test has been conducted.

11. Device according to any of claims 8 to 10, **characterized in that** a mechanical fuse (32) is provided for deactivating the means (31) for opening the clamp (2) when the opening of the door is effected at the moment the opening means are activated.

12. Device according to any of claims 4 to 11, **characterized in that** it furthermore comprises a volumetric pump, preferably a peristaltic pump, that is connected to the tube (1), the volumetric pump preferably being placed upstream of the clamp (2).
